# EUROPEAN PATENT SPECIFICATION

(11) **EP 1 482 029 B1**
(45) Date of publication and mention of the grant of the patent: **20.09.2006**
(21) Application number: 04380117.4
(22) Date of filing: 26.05.2004
(51) Int. Cl.: C12N 1/18, C12G 1/022, C12R 1/865

(54) **CECT 11774 and CECT 11775 Saccharomyces cerevisiae strains and their use in the production by alcoholic fermentation of alcoholic drinks and other foodstuff**
CECT 11774 und CECT 11775 Saccharomyces cerevisiae-Stämme und ihre Verwendung zur fermentativen Herstellung alkoholischer Getränke und anderer Nahrungsmittel
CECT 11774 et CECT 11775 souches de Saccharomyces cerevisiae et leur utilisation dans la production par fermentation de boissons alcooliques et d'autres produits alimentaires

(30) Priority: 27.05.2003 ES 200301245
(43) Date of publication of application: 01.12.2004
(73) Proprietor: Instituto Madrileno de Investigacion Agraria y Alimentari - IMIA, 28800 Alcala de Henares, Madrid (ES)
(72) Inventor: Hidalgo Togores, Pilar, 28800 Alcala de Henares (Madrid) (ES); Fernandez Oliva, Koro, 28800 Alcala de Henares (Madrid) (ES)
(74) Representative: Gonzalez Palmero, Fé

(56) References cited:
- ES-A- 2 089 982
- ES-A- 2 091 723
- ES-A- 2 180 397
- REGODON J A ET AL: "A simple and effective procedure for selection of wine yeast strains" FOOD MICROBIOLOGY (LONDON), vol. 14, no. 3, 1997, pages 247-254, XP002292505 ISSN: 0740-0020
- HIDALGO P ET AL: "[Enological profile and distribution of yeast strains of the "Vinos de Madrid" designation of origin.]" ALIMENTARIA, vol. 38, no. 327, November 2001 (2001-11), pages 121-126, XP009035266 ISSN: 0300-5755
- HIDALGO P ET AL: "TAXONOMIC STUDY OF THE YEAST FLORA OF MUSTS AND WINES FROM MADRID WINES" MICROBIOLOGIA (MADRID), vol. 7, no. 2, 1991, pages 120-125, XP001182689 ISSN: 0213-4101
- HIDALGO P: "Post-fermentation phase yeast flora in wines of the "Vinos de Madrid" designation of origin" ALIMENTARIA, vol. 34, no. 277, 1996, pages 101-109, XP009035265 ISSN: 0300-5755

## Description

### FIELD OF THE INVENTION

Generally, the invention is related to the production of yeasts of food and agriculture interest. Particularly, the invention is related to new yeast strains belonging to the *Saccharomyces cerevisiae* species, isolated from a microbiota native to the area belonging to the Guarantee of Origin and Quality of Wines of Madrid (Denominación de Origen Vinos de Madrid, D.O.), and applications thereof, especially, the use thereof in the production of alcoholic drinks obtained by fermentation, preferably wines.

### BACKGROUND OF THE INVENTION

Wine, beer and cider constitute illustrative examples of alcoholic drinks obtained by alcoholic fermentation of sugars, in which the *Saccharomyces* genus carries out a fundamental role.

Wine production can be carried out either by means of spontaneous fermentation of musts by wine yeasts found in the grape skin and in cellars, or by means of the use of inoculums of selected yeasts. The spontaneous fermentation creates problems of reproducibility in the quality of wines. Said problems can be corrected, at least in part, by means of the use of inoculums of selected yeasts, generally of the *Saccharomyces* genus, an alternative which allows for obtaining reproducible results in the quality of wines.

Although commercial active dry yeasts suitable for performing vinifications are known, their use is not always satisfactory due to problems of acclimatization to the environmental conditions of the wine-producing region, and/or to the lack of implantation or displacement by wild native yeasts, better acclimatized to the geo-botanical-climatological characteristics of said region. Furthermore, since foreign yeasts are concerned, they can confer different enological parameters to the wine, at least quantitatively.

Therefore, it is advisable to use yeasts native to the regions where they are going to be used in the vinification process, since said yeasts, generically denominated "selected local yeasts", generally have a better adaptation to the conditions of the wine-producing area and allow for ensuring the maintenance of their own characteristics typical of their wines. Moreover, the uniformization of the fermentative microbiota in different vinicultural areas is thus avoided.

The selection of local yeasts selected from the microbiota native to a wine-producing region is usually carried out by means of the comparative analysis of physicochemical and sensory parameters of the wines obtained therewith. Generally, those yeast strains typically belonging to the *Saccharomyces* genus, having suitable technological characteristics, such as high fermentative power, SO₂ resistance, low foaming, suitable action on malic acid, low production of volatile acidity, and suitable formation of other secondary volatiles, are selected. The experimental determination of these characteristics can be carried out following the methodology proposed by several authors [Caridi and Tini, (1991), Vignevini, 12, 62-66; Giudici and Zambonelli, (1992), Vignevini, 9, 29-34; Delteil and Lozano, (1995), Rev. Fr. Oenol. 153, 79-82; Delfini et al., (1995), Vignevini 4 (supplement), 12-15].

Numerous local yeasts selected from several Spanish wine-producing regions have been disclosed, for example, Alicante [Querol, A. et al., (1992), J. Food Sci. 57(1), 183-185], Bajo Miño [Angulo, L., et al., (1993), Viticultura/Enologia Profesional, 27, 28-36], Castilla-La Mancha [Spanish patent ES 2180397, Pérez-Coello, M. S., et al., (1999), Food Microbiol. 16, 563-573], El Penedés [Esteve-Zarzoso, B., et al., (2000), Food Microbiol. 17, 553-562], Extremadura [Spanish patent ES 2089982, Regodón, J. A., et al., (1996), Alimentaria, 269, 73-77], La Rioja [Gutiérrez, A. R., et al., (1997), Viticultura/Enologia Profesional, 51, 36-43], Navarra [De León, M. A., et al., (1994), Navarra Agraria, 86, 50-58], Valdepeñas [Spanish patent ES 2091723], etc.

Nevertheless, despite the existing demand for the sector, selected local yeasts native to the wine-producing region belonging to the Guarantee of Origin and Quality (D.O.) of Wines of Madrid have not been disclosed. Said selected local yeast strains native to the D.O. Wines of Madrid could be used by the wineries of said wine-producing region to control the fermentative process of the must, to ensure reproducibility thereof, to enhance the quality of the wines and to produce wines of a more homogeneous quality from one wine-harvest to another, maintaining typicality thereof, aspects required today by the consumer.

An ecological study has been carried out on the strains of *Saccharomyces cerevisiae* native to D.O. Wines of Madrid, determining their enological profile and distribution in the area, establishing the values of the enological characteristics most frequently found in yeasts [Hidalgo and Fernández (2001), Alimentaria 327, 121-126.] Within the parameters cited in the aforementioned study, strains with a sensitive phenotype predominate.

### DETAILED DESCRIPTION OF THE INVENTION

### A. IMIA 103 and IMIA 277 Strains

In one aspect, the invention is related to some selected *Saccharomyces cerevisiae* strains, native to the D.O. Wines of Madrid, identified as IMIA 103 and IMIA 277. Both cultures of said strains were deposited on 7 November, 2001, in the Spanish Type Culture Collection (Coleccion Española de Cultivos Tipo, CECT), Burjasot, Valencia, and were assigned access numbers CECT 1175 (IMIA 103) and CECT 11774 (IMIA 277).

IMIA 103 and IMIA 277 strains have been selected from 300 strains maintained in pure cultures, belonging to sub-areas of Arganda, Navalcarneno and San Martin de Valdeiglesias, which constitute the D.O. Wines of Madrid, after having been subjected to a sequential selection process on the basis of technological and quality parameters. The strains have been isolated by means of the consecutive decimal dilutions and plating technique, from samples collected in the final steps of the spontaneous fermentation of representative musts of said D.O., and have been characterized by their genetic profile and by their enological properties. Said IMIA 103 and IMIA 277 strains have an excellent and effective implantation rate in the fermentative medium compared with the native microbiota. In Example 1, the isolation and characterization of said IMIA 103 and IMIA 277 strains are described in detail.

The IMIA 103 and IMIA 277 strains have been characterized at the species level and they belong to the *Saccharomyces cerevisiae* species (Meyen ex Hansen, 1883) on the basis of the classification system proposed by Kreger-van Rij [The Yeasts, a taxonomic study. 3rd edition. Elsevier Science Publishers B.V., Amsterdam (1984)] and Kurtzman & Fell [The Yeasts, a taxonomic study. 4th edition. Elsevier Science, Amsterdam (1998)]. Likewise, the genetic profiles of said IMIA 103 and IMIA 277 strains have been obtained, following the methodology disclosed and developed by Pérez et al. [Lett. Appl. Microbiol., (2001), 33: 461-466].

The IMIA 103 and IMIA 277 strains have, among others, the characteristics indicated below.

### 1. Morphology

- Cellular morphology: oval, round cells vegetatively reproduced by multilateral budding in malt broth extract.
- In liquid medium (malt broth extract): they form sediment.
- In solid medium [agar yeast extract-malt extract (agar YM)]: light beige culture, creamy texture, smooth shiny surface, lobulated edges.
- In sporulation medium (Gorodkowa agar): ascospore formation (+), 1-4 spores per asca, round spores, persistent asca.
- In glucose-potato agar medium: formation of pseudomycelium (+), rudimentary.
- They do not grow in lysine agar medium.

### 2. Metabolism of various compounds

- Carbohydrate fermentation: in anaerobiosis conditions, both strains are glucose (+), sucrose (+), maltose (+), raffinose (+), lactose (-), melibiose (-), starch (-) ; while the IMIA 103 strain is galactose (+), and the IMIA 277 strain is galactose (-).
- Carbohydrate assimilation: in aerobiosis conditions, both strains are glucose (+), cellobiose (-), lactose (-), inositol (-), sucrose (+), maltose (+), raffinose (+).
- Nitrogenous compound assimilation: both strains are nitrate (-), ethylamine (-), cadaverine (-), lysine (-).
- Cycloheximide resistance: neither of the strains grows in the presence of 100 ppm of cycloheximide.
- Strong acid production from glucose: both strains are (-).
- Oxygen requirements: both strains are facultative anaerobes.
- Growth at different temperatures: the strains grow, at least, in the interval comprised between 15°C and 30°C.

### 3. Genetic profile of the IMIA 103 and IMIA 277 strains

The genetic profiles of the IMIA 103 and IMIA 277 strains have been obtained applying the technique of multiple amplification of 6 microsatellite *loci* (ScAAT1, ScAAT2, ScAAT3, ScAAT4, ScAAT5 and ScAAT6) by means of the polymerase chain reaction (PCR), disclosed and developed by Pérez et al. [Pérez et al., (2001) Lett. Appl. Microbiol., 33:461-466]. The genotype of the IMIA 103 and IMIA 277 strains for the 6 microsatellite *loci*, expressed in base pairs (bp), is shown in Table 1.

**Table 1**

| **Genotype of the IMIA 103 (CECT11775) and IMIA 277 (CECT 11774) strains, for the 6 microsatellite *loci*, expressed in base pairs** | | | | | | |
|---|---|---|---|---|---|---|
| | **ScAAT1** | **ScAAT2** | **ScAAT3** | **ScAAT4** | **ScAAT5** | **ScAAT6** |
| IMIA 103 | 221 | 369-384 | 247 | 301-328 | 216 | 255 |
| IMIA 277 | 188-236 | 375 | 250-346 | 301 | 219 | 249-255 |

### 4. Enological characteristics

As can be seen in detail in Example 1.2, the IMIA 103 and IMIA 277 strains have, among others, the following enological characteristics of industrial interest: high fermentative power, good fermentative energy, very good sugar/ethanol yield, very good sugar degradation capacity, high and regular fermentation rate, suitable sedimentation rate once the alcoholic fermentation is completed, low foaming, disperse culture in liquid medium, high SO₂ resistance, good ethanol tolerance, presence of the *killer* character, low production of volatile acidity and SO₂, very limited SH₂ production, very moderate degradation of malic acid, suitable glycerol production and limited nutritional requirements.

### 5. Implantation degree of the IMIA 103 and IMIA 277 strains in the fermentative medium

The IMIA 103 and IMIA 277 strains were tested as must fermentation initiating cultures on a pilot scale in an experimental winery, individually inoculating each one of them in several previously debourbaged white varietal musts. For comparison, spontaneous fermentations of the same musts without inoculation, serving as test samples, were also performed. Both strains optimally carried out the development of alcoholic fermentation, showing in all cases an excellent and effective implantation rate in the fermentative medium (Example 1.3), due to their high competitiveness degree compared with the native microbiota, despite that the dominant *S. cerevisiae* strains in the spontaneous fermentation had resistant phenotypes (neutral and killer phenotype).

### 6. Freeze tests

The IMIA 103 and IMIA 277 strains had a good viability after the freezing process (Example 1.4).

### 7. Dehydrated cultures

The IMIA 103 and TMIA 277 strains, subjected to lyophilization and subsequent rehydration processes, maintain their characteristics and have a high viability rate (Example 1.5). The IMIA 103 and IMIA 277 strains, in the lyophilized or dehydrated form, constitute an additional aspect of this invention.

### B. Biomass production of the IMIA 103 and IMIA 277 strains

In another aspect, the invention provides a process for obtaining a biomass of an *S. cerevisiae* strain chosen between the IMIA 103 (CECT 11775) strain and the IMIA 277 (CECT 11774) strain, which comprises cultivating said strain in a suitable culture medium under conditions allowing the growth thereof. Such conditions fall within the scope of knowledge of a person skilled in the art. In a particular embodiment, a biomass of said IMIA 103 and IMIA 277 strains can be obtained according to the process described in detail in Example 2. The use of said IMIA 103 and IMIA 277 strains in the biomass production constitutes an additional aspect of this invention.

### C. Applications

The IMIA 103 and IMIA 277 strains can generally be used in the food and agriculture industry. Illustratively, said strains can be used in the production of foodstuffs obtained by alcoholic fermentation, for example, bakery products (bread), fermented dairy products (kefir), etc., as well as in the production of alcoholic drinks obtained by fermentation, for example, wine, beer, cider etc, due to the fact that they optimally direct the alcoholic fermentation process.

Therefore, in another aspect, the invention is related to the use of said IMIA 103 and IMIA 277 strains in the production of alcoholic drinks obtained by fermentation. In a particular embodiment, the invention is related to the use of said IMIA 103 and IMIA 277 strains in the production of optionally modified wines, for example, white, rosé or red wines, by grape must fermentation of any of the *Vitis vinifera* variety, Spanish as well as foreign. Wines obtained by means of the use of said IMIA 103 and IMIA 277 strains are, due to the metabolism thereof, higher quality wines than those produced by means of spontaneous fermentation, and they have reproducible physicochemical and organoleptic characteristics. Specifically, the IMIA 103 strain is suitable for the production of white wines which have varietal aromas, enhancing the typicality, while the IMIA 277 strain is suitable for the production of white wines of neutral varieties by increasing the content of volatile compounds favorable for the aroma.

Said IMIA 103 and IMIA 277 strains can be used either separately or together.

The use of the IMIA 103 and IMIA 277 strains in the production of wines provides numerous advantages. Particularly, the use of said strains in wineries of the wine-producing region belonging to the D.O. Wines of Madrid allows for, among other things, (i) extending the range of local yeasts selected to carry out fermentations conducted in the vinification process, (ii) controlling the fermentative process of the must in order to make it less random since it does not depend on the yeasts naturally present in the grape skin and on the yeasts associated to the winery, preventing deviations due to the presence of unwanted yeasts which can alter the process and modify the product; (iii) producing wines in warm areas from the must of high chaptalization content and low nutrients; (iv) ensuring that the fermentation is really carried out by the inoculated yeast due to a suitable implantation of the selected yeasts in the fermentative medium, which yeasts act as biological agents responsible for the alcoholic fermentation of non-sterilized musts, for the purpose of achieving a result in the final product (circumstance of special relevance in warm regions where the implantation of selected yeasts during fermentation is a relevant problem because the dominance thereof is not ensured); (v) enhancing the quality of the wines; (vi) producing wines of more homogeneous quality from one grape harvest to another, maintaining typicality thereof; and (vii) producing wines with ecological practices.

The following Examples illustrate the invention but should not be considered limiting of the scope thereof.

### EXAMPLE 1

### Obtainment and characterization of the IMIA 103 and IMIA 277 strains

### 1.1 Isolation and characterization of the IMIA 103 and IMIA 277 strains

For obtaining the IMIA 103 and IMIA 277 strains, yeast strains belonging to the sub-areas of Arganda, Navalcarnero and San Martin de Valdeiglesias, which compose the D.O. Wines of Madrid, were tested. The musts were from the Malvar, Albillo, Airén, Tempranillo (or Tinto Fino) and Garnacha grape varieties, all of them authorized in the D.O. Wines of Madrid.

In brief, they were collected aseptically, and in sterile 500 mL bottles of must in fermentation for each one of the deposits to sample. Consecutive decimal dilutions of each sample were carried out in tubes with sterile physiological saline solution. 0.1 mL of each well-homogenized dilution was inoculated in Petri dishes with YM agar. After being incubated at 25°C for 72 hours, those dishes containing between 30-300 colony forming units (CFU) were chosen. Those CFU with a differentiated macroscopic morphology were chosen from each dish and were purified by consecutive re-platings in inclined YM agar. The pure cultures obtained (300 isolates) were stored in refrigeration. These strains were subjected to exclusive enological tests, among which tests were the determination of fermentative power, SO₂ resistance, foaming, action on malic acid, volatile acidity production, formation of secondary volatiles, etc. The strains identified as IMIA 103 and IMIA 277 deposited in the CECT with access numbers CECT 11775 and CECT 11774, respectively, were finally selected. Said IMIA 103 and IMIA 277 strains have the morphological, metabolic, physiological and genetic (genetic profile) characteristics mentioned in the description [characteristics 1-3 of section A of the "Detailed Description of the Invention"]. The enological characteristics of said strains are described below.

### 1.2. Enological characteristics of the IMIA 103 and IMIA 277 strains

The fermentative power, fermentative energy, sugar/ethanol yield, sugar degradation capacity, sedimentation rate, foaming and culture type, were determined carrying out microvinifications in bottles with sterile must of high chaptalization content, a frequent characteristic in warm areas. The bottles with must were inoculated in duplicate with each yeast strain and incubated at 25°C for approximately 25 days in semi-anaerobiosis conditions. The corresponding fermentative kinetics were assessed by means of daily weighing of the bottles until a constant value, carrying out the weight corrections with a test sample bottle (a non-inoculated must), determining the grams of CO₂ evolved per day. Once the alcoholic fermentation is completed, the alcohol content and residual reducing sugars were determined in the obtained wines (Community analyses applicable to the wine sector. EEC Rule 2676/90).

**The fermentative energy** of the IMIA 103 and IMIA 277 strains, expressed in g of CO₂ evolved in 48 hours, is 2.543 and 2.512, respectively.

Both strains have a **fermentative power** exceeding 14.0% by volume of ethanol, a sugar/ethanol yield close to 16.0 g/L of sugar to produce 1 alcohol content, they practically degrade the must sugars, leaving the wines dry (less than 3 g/L of residual sugars). Their fermentation rate is high and regular, foaming during the alcoholic fermentation is limited (1.0 cm of maximum height). The culture type, visually estimated, is disperse. The sedimentation rate expressed in the days passed since the must inoculation, in which a clear sedimentation of the cultures is observed, was between 9 and 14 days.

**SO**_{**2**} **resistance** was carried out in tubes containing 16 mL of YPD [yeast-peptone-dextrose] liquid medium adjusted to pH 3.8 and supplemented with concentrations of 50, 100, 150 and 200 mg of total SO₂/L, by means of addition of aliquots of a sterile solution of potassium metabisulfite (30 g/L). Likewise, tubes with the same medium in which SO₂ was not added, used as test samples (0 mg of SO₂/L), were prepared. The series of tubes containing 0, 50, 100, 150 and 200 mg of total SO₂/L were inoculated in duplicate with each yeast strain and hermetically sealed with a plastic stopper to prevent losses. They were incubated at 25°C for 16 days, after which the SO₂ resistance rate was determined, using the increase in the turbidity of the medium in the inoculated tubes in comparison with the test sample tubes, as indicative of growth, by means of UV/VIS spectrophotometry. The IMIA 103 and IMIA 277 strains resist 200 mg of total SO₂/L.

**The ethanol tolerance** was carried out in tubes containing 16 mL of YPD liquid medium, adjusted to pH 3.8, to which increasing amounts of sterile absolute ethanol were added up to a final concentration of 12.0 and 14-0% by volume. Likewise, tubes with the same medium but without ethanol, used as test samples (0.0% by volume of ethanol), were prepared. The series of tubes containing 0.0, 12.0 and 14.0% by volume of ethanol were inoculated in duplicate with each yeast strain and hermetically sealed with a plastic stopper to prevent losses. They were incubated at 25°C for 16 days, after which the ethanol tolerance degree was determined, using the increase in the turbidity of the medium in the inoculated tubes, compared with the test sample tubes, as indicative of growth, by means of UV/VIS spectrophotometry. The IMIA 103 and IMIA 277 strains tolerate ethanol, since they are able to grow in the presence of 12.0% by volume in the medium. The IMIA 277 strain even tolerates 14.0% by volume of ethanol.

**The production of hydrogen sulfide** (SH₂) was semi-quantitatively determined by means of carrying out microfermentations in flasks with a sterile synthetic medium, disclosed by Romano et al., (1985) [Romano P., Soli M.G., Suzzi G., Gracia L. and Zambonelli C. (1985). Appl, Environ. Microbiol. 50(4), 1064-1067], with modifications, prepared using YCB [11.7 g/L of Yeast Carbon Base (Difco)] supplemented with 40 g/L of glucose and 5 g/L of ammonium sulphate, in the presence of paper strips impregnated with lead acetate. The flasks were inoculated in duplicate with each strain under study and incubated at 25°C for 10 days, after which the darkening of the strips with lead acetate was assessed. For this, a subjective SH₂ production scale was carried out, using as extreme values those obtained in a very low producing reference strain (CECT 11133) and in a high producing reference strain (CECT 1375). The IMIA 103 and IMIA 277 strains are very poor SH₂ producers.

**The production of total SO**_{**2**} was determined from the samples obtained in the previous microfermentations (EEC Rule 2676/90). The IMIA 103 and IMIA 277 strains are low total SO₂ producers (17 and 15 mg of SO₂/L, respectively).

**The phenotype regarding the *killer* character** was determined using the methodology of Somers and Bevan (1969) [Somers J.M. and Bevan E.A. (1969). Genet. Res. Camb., 13, 71-83], with modifications. For this, two Petri dishes with YPD agar and methylene blue were used; at pH 4.2. One dish was spread plated with a *killer* reference strain (NCYC 738) and the other dish with a sensitive reference strain (NCYC 1006). The strains to be studied were streak plated on both dishes. After their incubation at 20°C for 72 hours, the growth and presence of an inhibitory halo in the dish with sensitive spread plating and only growth in the dish with *killer* spread plating, was clearly shown. The IMIA 103 and IMIA 277 strains show a *killer* phenotype, being producers of said toxin.

### 1.3 The implantation degree of the IMIA 103 and IMIA 277 strains in the fermentative medium

The implantation degree of the IMIA 103 and IMIA 277 strains in the fermentative medium was determined by collecting samples towards the end of the fermentation (density 1020), reflecting the representative microbiota of the fermentative process, both from the musts of the Airén and Malvar varieties, both individually inoculated with the IMIA 103 and IMIA 277 strains, and from their corresponding non-inoculated musts which served as test samples. From each sample, the isolation of the yeast strains present was carried out by means of the consecutive decimal dilutions and YM agar plating technique. After being incubated at 25°C for 72 hours, those dishes containing between 30 and 300 CFU were chosen, and 10 CFU per dish were randomly selected.

Genetic profile determination of the isolates was carried out by applying the microsatellite amplification technique by means of PCR, disclosed and developed by Gallego et al. (1998) (Am. J. Enol. Vitic. (1998), 49(3), 350-351). It is a very effective marker, since it is capable of detecting a high polymorphism among *S. cerevisiae* populations [Pérez et al., (2001) Lett. Appl. Microbiol., 33: 461-466].

The profile of 100% of the isolates obtained in the Airén and Malvar must samples inoculated with the IMIA 103 strains correspond to the 221 (bp) heterozygous genotype, typical for the IMIA 103 strain.

The profile of 100% of the isolates obtained in the Airén and Malvar must samples inoculated with IMIA 277 strains correspond to the 188-236 (bp) heterozygous genotype, typical for the IMIA 277 strain.

The profile of 100% of the isolates obtained in the Airén must samples without inoculation (test sample) correspond to the 242 (bp) heterozygous genotype, being different from those corresponding to the IMIA 103 and IMIA 277 strains.

The profile of the isolates obtained in the non-inoculated Malvar must samples (test sample) corresponds to: 40% to the 242 (bp) homozygote genotype, 20% to the 233 (bp) homozygote genotype, 20% to the 218 (bp) homozygote genotype, and 20% to the 203-233 (bp) heterozygote genotype, being different from those corresponding to the IMIA 103 and IMIA 277 strains in all cases.

The isolates with a 242 bp genotype have a *killer* phenotype, and the isolates with 233 (bp), 218 (bp) and 203-233 (bp) genotypes have a neutral phenotype.

### 1.4 Frozen cultures

The IMIA 103 and IMIA 277 strains have been subjected to freeze tests at -35°C and at -80°C for their storage, using YPD medium with glycerol as a cryoprotector. Said strains showed good viability after the freezing/thawing process.

### 1.5 Dehydrated (lyophilized) cultures

The IMIA 103 and IMIA 277 strains have been subjected to lyophilization tests, using skimmed and lactose milk as a cryoprotective agent. Dehydration by sublimation has been carried out in a Beta model 2-16 Christ lyophilizer with a two-phase program (main drying and final drying) at a working temperature of 80°C and drying chamber pressure of 0.250 and 0.050 mbar (100 Pa), respectively.

Taking into account that all the strains subjected to lyophilization processes loose a certain viability, the results obtained after lyophilization and subsequent rehydration process of both strains are very satisfactory. The strains maintain their characteristics and have a high viability rate (CFU/g), of the same order of magnitude as that obtained in another commercial yeast (*S. cerevisiae*), selected, used as a test sample.

### EXAMPLE 2

### Biomass obtainment

A biomass of the IMIA 103 and IMIA 277 strains was obtained from standardized pre-inoculums, in their respective fermentators (Biostat A) of 2.5 L capacity, with control of pH, temperature, dissolved oxygen, stirring and time, physicochemical factors affecting cell growth. The conditions were as follows:
- Volume: 1.5 L of YPD medium
- Pre-inoculum: all the fermentators were inoculated with the pre-inoculum volume necessary for starting from an O.D. = 0.1 (measure in a spectrophotometer at λ=600 nm).
- pH: was maintained at a constant value of 6.0 due to the addition of sodium hydroxide (1M) and orthophosphoric acid solutions (17%).
- Temperature: was maintained constant at a value of 30°C.
- Dissolved oxygen: was maintained at 30% by means of automatic control first acting at the air inlet valve opening and, in the event that the air flow rate is insufficient, by increasing the stirring (established maximum of 800 rpm).
- Stirring: an initial stirring of 300 rpm was maintained.
- Time: 20 hours.

During the process, culture homogeneity was checked by means of direct observation at the optical microscope, and, after its completion, the strain viability (CFU/mL) was determined.

Obtaining a biomass is rapid and homogeneous, showing a high viability corresponding to the same order of magnitude as that obtained in another commercial yeast (*S. cerevisiae*) selected, used as a test sample. In any case, the IMIA 103 and IMIA 277 strains have the advantage of consuming a lower amount of oxygen for their multiplication than the test sample yeast.

### EXAMPLE 3

### Chemical and sensory characteristics of the wines produced using the IMIA 103 and IMIA 277 strains

The chemical and sensory characteristics of white wines produced with the IMIA 103 and IMIA 277 strains have been analyzed (separately). The productions of white wines with the IMIA 103 and IMIA 277 strains, used as previously debourbaged initiating cultures of must (Airén and Malvar) fermentation , have been carried out at a controlled temperature (18-20°C).

Applying a sufficient plating (2-3 x 10⁶CFU/mL), the beginning of fermentation is rapid with a very short latency period. The fermentation rate is very regular, giving rise to a limited, fine and homogeneous foaming. The sugar/ethanol yield of both strains is very good.

Both strains (IMIA 103 and IMIA 277) have given rise to wines with low volatile acidity and residual reducing sugar contents, very moderately degrading malic acid. Glycerol production is suitable and SH₂ production is not detected.

In all the cases, the characteristics of the wines have been better than those of wines obtained by means of spontaneous fermentation of the same non-inoculated starting musts, which served as test samples.

Among the analyzed volatile compounds (esters, alcohols, fatty acids, etc), the formation of foreign compounds has not been detected. The volatile compounds which can negatively affect the quality of the wine (acetaldehyde, ethyl acetate), have been found in low concentration, in any case being able to contribute to the olfactory complexity thereof.

Sensory analysis of the wines produced with the IMIA 103 and IMIA 277 strains was carried by a qualified wine tasters panel, using a penalization tasting record, according to which a lower score corresponds to higher valued wines. The scoring interval is comprised between 0 and 90 points, both inclusive, for wines that were not eliminated. A score exceeding 90 implies the elimination of the wines. The assessments carried out were: visual phase, olfactory phase (intensity and quality), gustative phase (intensity and quality), and harmony of the total impression. The overall final score is the result of the rating means for each wine, not taking into account the highest and lowest marks, to eliminate abnormal scores. The obtained results are shown in the Table 2.

**Table 2**

| **Overall wine scoring** | | |
|---|---|---|
| **STRAIN** | **OVERALL WINE SCORING** | |
| | **Airén** | **Malvar** |
| **IMIA 103** | **45.50** | **36.20** |
| **IMIA 277** | **38.00** | **38.20** |
| **TEST SAMPLE** | **48.60** | **44.00** |

The obtained results show that the white wines produced with the IMIA 103 and IMIA 277 strains rated better than those produced by means of spontaneous fermentation. Due to their own metabolism, both strains have given rise to high quality wines.

The wines obtained with the IMIA 103 and IMIA 277 strains are pallid, harmonious, fresh and fruity, having a higher olfactory intensity and gustative persistence than those produced by means of spontaneous fermentation.

The wine produced with the Airén variety which obtained the best score was the one in which the IMIA 277 strain was used, as a fermentation initiating culture, increasing the contents of compounds favorable for the aroma.

The wine produced with the Malvar variety which obtained the best score was the one in which the IMIA 103 strain was used, as a fermentation initiating culture, showing varietal aromas.

## Claims

1. A *Saccharomyces cerevisiae* strain selected between the strain identified as IMIA 103 and the strain identified as IMIA 277, deposited in the CECT with access numbers CECT 11775 and CECT 11774, respectively.

2. A strain according to claim 1, in a lyophilized or dehydrated form.

3. The use of a *Saccharomyces cerevisiae* strain according to claim 1, selected between the strain identified as IMIA 103 (CECT 11775), the strain identified as IMIA 277 (CECT 11774) and mixtures thereof, in the production of an alcoholic drink obtained by alcoholic fermentation.

4. The use according to claim 3, wherein said drink is selected among wine, beer and cider.

5. The use according to claim 3, wherein said alcoholic drink is selected among white, rosé or red wines, optionally modified, produced from grape musts of a *Vitis vinifera* variety, Spanish as well as foreign.

6. The use of the *S. cerevisiae* strain according to claim 3 identified as IMIA 103 (CECT 11775) in the production of white wines of aromatic grape varieties.

7. The use of the *S. cerevisiae* strain according to claim 3 identified as IMIA 277 (CECT 11774) in the production of white wines of neutral grape varieties.

8. The use of a *Saccharomyces cerevisiae* strain according to claim 1 selected between the strain identified as IMIA 103 (CECT 11775), the strain identified as IMIA 277 (CECT 11774) and mixtures thereof, in the production of a foodstuff obtained by alcoholic fermentation.

9. The use according to claim 8, wherein said foodstuff obtained by alcoholic fermentation is selected between bread and a fermented dairy product.

10. The use of a *Saccharomyces cerevisiae* strain according to claim 1 selected between the strain identified as IMIA 103 (CECT 11775), the strain identified as IMIA 277 (CECT 11774) and mixtures thereof, in the biomass production.

## Patentansprüche

1. Ein *Saccharomyces cerevisiae* Stamm, ausgewählt aus dem als IMIA 103 identifizierten Stamm und dem als IMIA 277 identifizierten Stamm, die in der CECT mit den Zugangsnummern CECT 11775 bzw. CECT 11774 deponiert sind.

2. Ein Stamm gemäß Anspruch 1 in gefriergetrockneter oder entwässerter Form.

3. Verwendung eines *Saccharomyces cerevisiae* Stammes gemäß Anspruch 1, ausgewählt aus dem als IMIA 103 (CECT 11775) identifizierten Stamm, dem als IMIA 277 (CECT 11774) identifizierten Stamm und deren Mischungen, in der Herstellung eines durch alkoholische Gärung erhaltenen alkoholischen Getränkes.

4. Verwendung gemäß Anspruch 3, wobei besagtes Getränk aus Wein, Bier und Apfelwein ausgewählt ist.

5. Verwendung gemäß Anspruch 3, wobei besagtes alkoholisches Getränk aus wahlweise modifizierten Weiß-, Rose- oder Rotweinen ausgewählt ist, die aus Weinmost einer sowohl spanischen als auch ausländischen Sorte von *Vitis vinifera* hergestellt wurden.

6. Verwendung des als IMIA 103 (CECT 11775) identifizierten S. *cerevisiae* Stammes gemäß Anspruch 3 in der Herstellung von Weißweinen aus aromatischen Traubensorten.

7. Verwendung des als IMIA 277 (CECT 11774) identifizierten S. *cerevisiae* Stammes gemäß Anspruch 3 in der Herstellung von Weißweinen aus neutralen Traubensorten.

8. Verwendung eines *Saccharomyces cerevisiae* Stammes gemäß Anspruch 1, ausgewählt aus dem als IMIA 103 (CECT 11775) identifizierten Stamm, dem als IMIA 277 (CECT 11774) identifizierten Stamm und deren Mischungen, in der Herstellung eines durch alkoholische Gärung erhaltenen Nahrungsmittels.

9. Verwendung gemäß Anspruch 8, wobei besagtes, durch alkoholische Gärung erhaltene Nahrungsmittel aus Brot und einem gegorenen Milchprodukt ausgewählt ist.

10. Verwendung eines *Saccharomyces cerevisiae* Stammes gemäß Anspruch 1, ausgewählt aus dem als TMIA 103 (CECT 11775) identifizierten Stamm, dem al IMIA 277 (CECT 11774) identifizierten Stamm und deren Mischungen, in der Herstellung von Biomasse.

## Revendications

1. Une souche de *Saccharomyces cerevisiae* choisie entre la souche identifiée comme IMIA 103 et la souche identifiée comme IMIA 277, déposées à la CECT (Collection Espagnole de Cultures Type) sous les numéros d'accès CECT 11775 et CECT 11774, respectivement.

2. Une souche selon la revendication 1, sous une forme lyophilisée ou déshydratée.

3. L'utilisation d'une souche de *Saccharomyces cerevisiae* selon la revendication 1, choisie entre la souche identifiée comme IMIA 103 (CECT 11775), la souche identifiée comme IMIA 277 (CECT 11774) et leurs mélanges, dans la production d'une boisson alcoolisée obtenue par fermentation alcoolique

4. L'utilisation selon la revendication 3, où ladite boisson est choisie parmi le vin, la bière et le cidre.

5. L'utilisation selon la revendication 3, où ladite boisson alcoolisée est choisie parmi des vins blancs, rosés ou rouges, facultativement modifiés, produits à partir de moûts de raisins d'une variété Vitis vinifera, espagnols ou étrangers.

6. L'utilisation de la souche de *S. cerevisiae* selon la revendication 3 identifiée comme IMIA 103 (CECT 11775) dans la production de vins blancs de cépages aromatiques.

7. L'utilisation de la souche de *S. cerevisiae* selon la revendication 3 identifiée comme IMIA 277 (CECT 11774) dans la production de vins blancs de variétés de raisins neutres.

8. L'utilisation d'une souche de *Saccharomyces cerevisiae* selon la revendication 1, choisie entre la souche identifiée comme IMIA 103 (CECT 11775), la souche identifiée comme IMIA 277 (CECT 11774) et leurs mélanges, dans la production d'une denrée alimentaire obtenue par fermentation alcoolique.

9. L'utilisation selon la revendication 8, où ladite denrée alimentaire obtenue par fermentation alcoolique est choisie entre le pain et un produit laitier fermenté.

10. L'utilisation d'une souche de *Saccharomyces cerevisiae* selon la revendication 1, choisie entre la souche identifiée comme IMIA 103 (CECT 11775), la souche identifiée comme IMIA 277 (CECT 11774) et leurs mélanges, dans la production de biomasse.
